# NEUE EUROPÄISCHE PATENTSCHRIFT

(11) **EP 2 612 609 B2**
(45) Veröffentlichungstag und Bekanntmachung des Hinweises auf die Entscheidung über den Einspruch: **27.01.2021**
(45) Hinweis auf die Patenterteilung: 27.04.2016
(21) Anmeldenummer: 12008531.1
(22) Anmeldetag: 21.12.2012
(51) Int. Cl.: A61B 17/29

(54) **Medizinisches Instrument**
Medical instrument
Instrument médical

(30) Priorität: 04.01.2012 DE 102012200073
(43) Veröffentlichungstag der Anmeldung: 10.07.2013
(73) Patentinhaber: Karl Storz SE & Co. KG, 78532 Tuttlingen (DE)
(72) Erfinder: Kärcher, Daniel, 78532 Tuttlingen (DE); Stefan, Jochen, 78532 Tuttlingen (DE)
(74) Vertreter: Wimmer, Stephan

(56) Entgegenhaltungen:
- WO-A1-2008/005433
- WO-A1-2009/046490
- DE-A1-102004 025 041
- DE-A1-102006 038 515
- JP-A- H1 099 340
- JP-A- H07 265 326
- US-A1- 2008 154 299

## Beschreibung

Die vorliegende Erfindung ist auf ein medizinisches Instrument mit einem Schaft, einer Handhabungseinrichtung am proximalen Ende des Schafts und einem im Schaft bewegbaren Übertragungselement bezogen. Insbesondere ist die vorliegende Erfindung auf eine Kombinierbarkeit einer Handhabungseinrichtung mit unterschiedlich ausgebildeten Übertragungselementen bezogen.

Die Vorschriften für die Reinigung und Sterilisierung wiederverwendbarer medizinischer, insbesondere chirurgischer, Instrumente stellen hohe Anforderungen, die eine Zerlegbarkeit der chirurgischen Instrumente erfordert. Insbesondere können in der Regel der Schaft und die Handhabungseinrichtung voneinander getrennt werden. Die Handhabungseinrichtung weist in der Regel mindestens zwei relativ zueinander bewegbare Griffteile auf, von denen eines mit dem Schaft und das andere mit einer im Schaft angeordneten Übertragungsstange gekoppelt ist. Eine Bewegung der beiden Griffteile relativ zueinander hat eine Bewegung der Übertragungsstange im Schaft zur Folge.

Das proximale Ende der Übertragungsstange ist insbesondere als Kupplungselement zur lösbaren mechanischen Kopplung mit einem Griffteil der Handhabungseinrichtung ausgebildet. Bei unterschiedlichen Schaftdurchmessern werden Übertragungsstangen mit unterschiedlichen Querschnitten verwendet. Insbesondere wenn die Übertragungsstange mit einem vom Schaft trennbaren Werkzeug verbunden ist und deshalb nach distal aus dem Schaft herausgezogen werden kann, ist die Größe des Kupplungselements am proximalen Ende der Übertragungsstange vom Querschnitt des Schafts abhängig.

Bei medizinischen Instrumenten für mikroinvasive Eingriffe geht ein Trend zu immer dünneren Schäften. Dies erfordert immer kleinere Kupplungselemente an den proximalen Enden der Übertragungsstangen. Herkömmlich baugleich für viele verschiedene medizinische Instrumente verwendete Handhabungseinrichtungen sind deshalb nicht mehr unverändert verwendbar. Stattdessen müssen die Handhabungseinrichtungen modifiziert und im ungünstigsten Fall immer wieder an immer kleinere Kupplungselemente an proximalen Enden von Übertragungsstangen angepasst werden.

Die Offenlegungsschrift DE 10 2004 025 41 A1 offenbart eine Laparoskopie-Zange, deren Griff ein festes und ein bewegliches Griffstück umfasst. Das feste Griffstück kann ein Schaftrohr mit einer Betätigungsstange für ein bewegliches Schneidinstrument aufnehmen. Die Bewegung des beweglichen Griffstücks wird mittels eines Schiebers übertragen, der in einer Aufnahme des festen Griffstücks verbleibt. Dieser Schieber weist eine Spannzange auf, welche das proximale Ende der Betätigungsstange festhält und an dessen Durchmesser bzw. Geometrie angepasst ist. Es können nur Schaftrohre bzw. Betätigungsstangen montiert werden, die dem Handgriff bzw. Schieber angepasst sind.

Die Offenlegungsschrift WO 2009/046490 A1 lehrt ein zerlegbares medizinisches Instrument. Das proximale Ende der Betätigungsstange zur Betätigung des Instruments wird von federbelasteten Klauen gehalten. Diese befinden sich in einer zylindrischen Kapsel, welche durch über eine Zahnstangenordnung mit einem beweglichen Griffteil verbunden ist. Auf diese Weise kann das Werkzeug am distalen Ende des Instrumentenschafts geöffnet und geschlossen werden. Die Funktionssicherheit ist nur sichergestellt, wenn Schaft und Betätigungsstange maßlich zusammenpassen, sodass unterschiedlich starke Werkzeuge nur bedingt verwendet werden können.

Eine Aufgabe der vorliegenden Erfindung besteht darin, ein verbessertes medizinisches Instrument zu schaffen und eine Kombination unterschiedlicher Schäfte und unterschiedlicher Übertragungselemente mit ein und derselben Handhabungseinrichtung, insbesondere mit einer herkömmlichen Handhabungseinrichtung, zu ermöglichen.

Diese Aufgabe wird durch die Gegenstände der unabhängigen Ansprüche gelöst.

Weiterbildungen sind in den abhängigen Ansprüchen angegeben.

Ausführungsbeispiele der vorliegenden Erfindung beruhen auf der Idee, einen Übertragungsadapter vorzusehen, der einerseits mit dem proximalen Ende eines Übertragungselements in einem Schaft für ein medizinisches Instrument und andererseits mit einer Handhabungseinrichtung für ein medizinisches Instrument koppelbar ist. Insbesondere ist für eine Mehrzahl unterschiedlicher Übertragungselemente bzw. für eine Mehrzahl von Übertragungselementen mit unterschiedlich ausgebildeten Kupplungen an ihren proximalen Enden jeweils ein Übertragungsadapter vorgesehen. Ein distales Ende bzw. eine Kupplung am distalen Ende des Übertragungsadapters ist jeweils an eine bestimmte Ausgestaltung des proximalen Endes eines Übertragungselements angepasst, die proximalen Enden der Übertragungsadapter sind gleich und insbesondere an eine Standard-Handhabungseinrichtung angepasst.

Ein Übertragungsadapter für ein medizinisches Instrument mit einem Schaft, einem im Schaft bewegbaren Übertragungselement und einer Handhabungseinrichtung mit einem ersten Teil, das mit einem proximalen Ende des Schafts mechanisch starr koppelbar ist, und einem zweiten Teil, das gegenüber dem ersten Teil bewegbar ist, umfasst eine erste Kupplung zur lösbaren mechanischen Kopplung mit dem zweiten Teil der Handhabungseinrichtung und eine zweite Kupplung zur lösbaren formschlüssigen mechanischen Kopplung mit einem proximalen Ende des Übertragungselements, wobei der Übertragungsadapter in einen proximalen Bereich des Schafts derart integriert ist, dass er nicht zerstörungsfrei oder nicht ohne Verwendung von Werkzeug aus dem Schaft entnommen werden kann.

Der Übertragungsadapter ist insbesondere Bestandteil eines medizinischen Instruments oder mit anderen Bestandteilen zusammen zur Bildung eines medizinischen Instruments vorgesehen und ausgebildet. Der Schaft ist gerade oder gekrümmt, teilweise oder vollständig starr oder teilweise oder vollständig elastisch oder plastisch verformbar. Das Übertragungselement ist im Schaft translatorisch und/oder rotatorisch bewegbar, um eine Zug- und/oder Druckkraft und/oder ein Drehmoment zwischen der Handhabungseinrichtung und einem distalen Ende des Schafts, insbesondere einem Werkzeug am distalen Ende des Schafts, zu übertragen.

Der erste Teil und der zweite Teil der Handhabungseinrichtung sind insbesondere ähnlich wie die Griffteile einer Schere ausgebildet und mit den Fingern einer Hand relativ zu einander bewegbar. Alternativ sind der erste Teil und der zweite Teil der Handhabungseinrichtung ähnlich wie die Griffteile einer Zange ausgebildet. Alternativ weist die Handhabungseinrichtung ein drittes Teil auf, das insbesondere symmetrisch zum zweiten Teil der Handhabungseinrichtung ausgebildet und angeordnet ist, wobei der zweite Teil und der dritte Teil der Handhabungseinrichtung zur lösbaren und gemeinsamen mechanischen Kopplung mit der ersten Kupplung des Übertragungsadapters ausgebildet sind. Diese Ausgestaltung der Handhabungseinrichtung kann der von Nadelhaltern bekannten Bauart ähneln.

Die Handhabungseinrichtung und insbesondere deren erster Teil sind beispielsweise für eine lösbare Rastverbindung mit dem proximalen Ende eines Schafts ausgebildet. Der zweite Teil der Handhabungseinrichtung ist insbesondere durch ein Gelenk (beispielsweise Welle oder Festkörpergelenk) mit dem ersten Teil verbunden und gegenüber diesem um eine Schwenkachse senkrecht zur Längsachse eines mit der Handhabungseinrichtung zu verbindenden Schafts schwenkbar.

Die erste Kupplung ist insbesondere am proximalen Ende des Übertragungsadapters angeordnet und für eine gelenkige mechanische Kopplung mit dem zweiten Teil der Handhabungseinrichtung ausgebildet. Die zweite Kupplung ist insbesondere am distalen Ende des Übertragungsadapters angeordnet. Wie das Übertragungselement ist auch der Übertragungsadapter zur Übertragung einer Kraft und/oder eines Drehmoments von dem zweiten Teil der Handhabungseinrichtung zum proximalen Ende des Übertragungselements ausgebildet.

Die zweite Kupplung des Übertragungsadapters kann zur formschlüssigen Kupplung mit einem proximalen Ende eines beliebigen Übertragungselements ausgebildet sein. Der Übertragungsadapter kann an ein beliebiges Übertragungselement und an eine beliebige Handhabungseinrichtung angepasst sein und damit eine Kombination eines beliebigen Übertragungselements mit einer beliebigen Handhabungseinrichtung ermöglichen. Insbesondere können immer dünnere Schäfte und entsprechend immer dünnere Übertragungselemente mit immer kleineren Kupplungselementen an ihren proximalen Enden mit einer herkömmlichen Handhabungseinrichtung, insbesondere mit einer Standard-Handhabungseinrichtung, kombiniert werden. Damit entfallen Entwicklungskosten und Kosten für neue Werkzeuge für die Herstellung von Handhabungseinrichtungen, die an verschiedene Übertragungselemente angepasst sind. Durch eine geringe Typenvielfalt und größere Stückzahlen sinken die Fertigungs-Stückkosten und der Aufwand für Lagerhaltung und Logistik.

Ferner ist die zweite Kupplung des Übertragungsadapters insbesondere für eine lösbare, aber starre mechanische Kupplung mit dem proximalen Ende einer Übertragungsstange ausgebildet. Durch eine starre, nicht-gelenkige Kopplung entfallen Verschleiß und mechanisches Spiel, die bei einer gelenkigen Ausbildung nahezu zwangsläufig auftreten bzw. vorliegen. Dies kann eine weitere Miniaturisierbarkeit von Übertragungselementen und deren proximalen Enden ermöglichen oder vereinfachen. Die erste Kupplung kann deutlich größer als das proximale Ende eines Übertragungselements ausgebildet und damit in deutlich geringerem Maße anfällig für Verschleiß sein und ein größeres mechanisches Spiel ermöglichen.

Bei einem Übertragungsadapter, wie er hier beschrieben ist, umfasst die zweite Kupplung insbesondere eine bewegbare Greifbacke zum form- oder kraftschlüssigen Halten des proximalen Endes eines Übertragungselements.

Die zweite Kupplung kann zwei oder mehr bewegbare Greifbacken umfassen. Insbesondere umfasst die zweite Kupplung eine feststehende Greifbacke und eine bewegbares Greifbacke oder zwei gegenläufig bewegbare Greifbacken.

Die bewegbare Greifbacke ist insbesondere um eine Schwenkachse schwenkbar.

Die Schwenkbarkeit der Greifbacke wird beispielsweise durch ein entsprechendes Lager oder ein Festkörpergelenk erzeugt.

Die bewegbare Greifbacke ist insbesondere entlang eines vorbestimmten Pfads bewegbar.

Insbesondere ist eine Führung mittels einer Kulisse in Form eines Schlitzes, einer Nut oder eines Stegs vorgesehen, wobei eine Gleitfläche den Pfad definiert. Die Greifbacke kann gleichzeitig um eine Schwenkachse schwenkbar und entlang eines vorbestimmten Pfads bewegbar sein. Insbesondere ist die Greifbacke um eine Schwenkachse schwenkbar, wobei ein von der Schwenkachse beabstandeter Bereich der Greifbacke mittels der Kulisse geführt ist.

Eine oder mehrere Greifbacken können eine einfach herstellbare und gleichzeitig zuverlässige, robuste und präzise lösbare mechanische Kopplung des Übertragungsadapters mit einem proximalen Ende gegebener Geometrie eines Übertragungselements ermöglichen.

Ein Übertragungsadapter mit einer bewegbaren Greifbacke, wie er hier beschrieben ist, umfasst insbesondere ferner ein elastisches Element, das vorgesehen und ausgebildet ist, um die bewegbare Greifbacke zu einer das proximale Ende eines Übertragungselements greifenden Position hin mechanisch vorzuspannen.

Das elastische Element ist insbesondere derart mit der bewegbaren Greifbacke gekoppelt, dass eine Auslenkung der bewegbaren Greifbacke aus der greifenden Position eine Verformung des elastischen Elements bewirkt, die eine rückstellende Kraft und/oder ein rückstellendes Drehmoment zur Folge hat, um die bewegbare Greifbacke wieder in die greifende Position zu bewegen. Das elastische Element kann mit der bewegbaren Greifbacke und/oder einem anderen Teil des Übertragungsadapters einstückig ausgebildet sein. Das elastische Element weist insbesondere die Gestalt einer Blattfeder oder einer anderen Feder auf. Alternativ kann das elastische Element in Gestalt eines O-Rings oder eines anderen Körpers aus einem Elastomer oder einem anderen elastischen Material ausgebildet sein und bei einer Auslenkung der bewegbaren Greifbacke aus der greifenden Position auf gedehnt, gestaucht, geschert und/oder tordiert werden.

Das elastische Element kann ausgebildet sein, um ohne weitere Maßnahmen eine hinreichend starke mechanische Kopplung zwischen der bewegbaren Greifbacke und dem proximalen Ende eines Übertragungselements zu ermöglichen. Alternativ können weitere Maßnahmen vorgesehen sein, um eine mechanische Kopplung zwischen der bewegbaren Greifbacke und dem proximalen Ende eines Übertragungselements zu unterstützen oder zu verriegeln.

Bei einem Übertragungsadapter, wie er hier beschrieben ist, weist insbesondere die zweite Kupplung ein rotierbares Kupplungsglied auf, wobei das rotierbare Kupplungsglied zwischen einer ersten Positionen, in der eine mechanische Kopplung mit dem proximalen Ende des Übertragungselements herstellbar und lösbar ist, und einer zweiten Position, in der eine mechanische Kopplung mit dem proximalen Ende des Übertragungselements verriegelt ist, rotierbar ist.

Das rotierbare Kupplungsglied ist insbesondere um eine Rotationsachse parallel zu einer Längsachse des Übertragungselements rotierbar. Dabei ist die Rotationsachse des rotierbaren Kupplungsglieds insbesondere von der Längsachse des Übertragungselements beabstandet. Alternativ kann das Kupplungsglied um eine Rotationsachse senkrecht zu einer Längsachse des Übertragungselements rotierbar sein. Unabhängig von einer Anordnung der Rotationsachse parallel oder senkrecht zur Längsachse eines mit dem Kupplungsglied zu koppelnden Übertragungselements oder in einer anderen Orientierung der Rotationsachse weist das rotierbare Kupplungsglied insbesondere eine Ausnehmung oder eine Öffnung zum Aufnehmen des proximalen Endes des Übertragungselements. Die Öffnung am rotierbaren Kupplungsglied weist ein erstes Ende und ein zweites Ende auf. Das erste Ende der Öffnung ist so ausgebildet, dass in der ersten Position des rotierbaren Kupplungsglieds das proximale Ende des Übertragungselements am ersten Ende der Öffnung in die Öffnung aufgenommen und aus ihr entnommen werden kann. Das zweite Ende der Öffnung ist so ausgebildet, dass in der zweiten Position des rotierbaren Kupplungsglieds das proximale Ende des Übertragungselements am zweiten Ende der Öffnung in der Öffnung gehalten wird. Insbesondere weist die Öffnung am ersten Ende eine große Breite oder einen großen Querschnitt und am zweiten Ende eine kleine Breite oder einen kleinen Querschnitt auf. Ein Beispiel ist eine schlüssellochförmige Gestalt der Öffnung.

Alternativ kann die zweite Kupplung am Übertragungsadapter für eine lösbare mechanische Kopplung ausgebildet sein, die durch eine relative Rotation von Übertragungselement und zweiter Kupplung um eine Achse parallel zur Längsachse des Übertragungselements hergestellt oder verriegelt und gelöst oder entriegelt werden kann.

Alternativ kann die zweite Kupplung am Übertragungsadapter als Rastverbindung zum proximalen Ende eines Übertragungselements ausgebildet sein. Insbesondere ist eine gefederte Raste vorgesehen, die ausgebildet ist, um in eine Öffnung, Ausnehmung oder Hinterschneidung am proximalen Ende eines Übertragungselements einzugreifen. Die Rastverbindung mittels der gefederten Raste kann durch Betätigung eines Druccknopfes oder einer anderen Einrichtung manuell lösbar sein.

Ein Übertragungsadapter, wie er hier beschrieben ist, ist insbesondere in einen proximalen Bereich eines Schafts integriert.

Insbesondere ist der Übertragungsadapter in den Abschnitt eines Schafts integriert, der zur Anordnung in einer entsprechenden Ausnehmung in einer Handhabungseinrichtung vorgesehen und ausgebildet ist. Der Übertragungsadapter bzw. sein Raumbedarf erfordert deshalb keine Vergrößerung des Querschnitts des Schafts. Ferner ist am proximalen Ende des Schafts eine Verunreinigung des Übertragungsadapters während des Gebrauchs des Schafts unwahrscheinlich. Dadurch wird die Reinigung des Schafts mit dem Übertragungsadapter vereinfacht.

Bei einem Übertragungsadapter, wie er hier beschrieben ist, ist die zweite Kupplung insbesondere ausgebildet, um verriegelt zu sein, wenn der Übertragungsadapter in einer Handhabungseinrichtung angeordnet ist.

Die zweite Kupplung ist insbesondere ausgebildet, um in dem Sinne verriegelt zu sein, dass sie nicht geöffnet werden kann bzw. eine Entkopplung nicht möglich ist, wenn der Übertragungsadapter in der Handhabungseinrichtung angeordnet ist. Eine mechanische Kopplung zwischen dem Übertragungsadapter und dem proximalen Ende eines Übertragungselements ist deshalb nur möglich, solange der Übertragungsadapter außerhalb einer Handhabungseinrichtung angeordnet ist. Die Verriegelung der zweiten Kupplung ermöglicht bei Einhaltung einer vorbestimmten Reihenfolge des Zerlegens bzw. Zusammensetzens von Bestandteilen eines medizinischen Instruments eine zuverlässige mechanische Kupplung des Übertragungsadapters mit dem proximalen Ende eines Übertragungselements. Insbesondere kann ein medizinisches Instrument mit dem Übertragungsadapter so ausgebildet sein, dass beim Zerlegen und Zusammensetzen die gleichen Handgriffe in der gleichen Reihenfolge erforderlich sind, die auch bei einem herkömmlichen Instrument auszuführen sind, dessen Werkzeug mit dem distalen Ende des Übertragungselements dauerhaft verbunden ist.

Bei einem Übertragungsadapter, wie er hier beschrieben ist, weist das proximale Ende des Übertragungsadapters insbesondere einen kugelflächenförmigen Oberflächenabschnitt auf.

Insbesondere weist das proximale Ende des Übertragungsadapters im Wesentlichen die Gestalt einer Kugel auf. Damit kann der Übertragungsadapter eine Kombination eines beliebigen Übertragungselements mit einer Standard-Handhabungseinrichtung ermöglichen, bei einem Durchmesser von 2,5 mm beispielsweise mit einer Handhabungseinrichtung aus dem unter der Bezeichnung "Clickline" angebotenen Produktprogramm der Anmelderin.

Ein Schaft für ein medizinisches Instrument umfasst ein proximales Ende zur Kopplung mit einem ersten Teil einer Handhabungseinrichtung, ein distales Ende zur Kopplung mit einem Werkzeug und einen Übertragungsadapter, wie er hier beschrieben ist, der relativ zum Schaft bewegbar ist.

Insbesondere ist der Übertragungsadapter innerhalb des Schafts und an dessen proximalem Ende angeordnet und relativ zum Schaft translatorisch und/oder rotatorisch bewegbar. Die Integration des Übertragungsadapters in einen Schaft, insbesondere in einen proximalen Bereich des Schafts, der zur Anordnung in einer Handhabungseinrichtung vorgesehen und ausgebildet ist, kann eine gegenüber herkömmlichen medizinischen Instrumenten völlig unveränderte Verwendung bzw. Handhabung ermöglichen. Da in der Regel jeder Schaft für ein Übertragungselement eines vorbestimmten Querschnitts ausgebildet ist, kann der Übertragungsadapter im Schaft ohne Weiteres an das proximale Ende des Übertragungselements angepasst sein.

Der Übertragungsadapter ist so in den Schaft integriert, dass er nicht zerstörungsfrei oder nicht ohne Verwendung von Werkzeug aus dem Schaft entnommen und deshalb auch nicht verloren werden kann und im Schaft vor mechanischer Beschädigung geschützt ist.

Ein Schaftadapter umfasst ein proximales Ende zur Kopplung mit einem ersten Teil einer Handhabungseinrichtung, ein distales Ende zur Kopplung mit einem proximalen Ende eines Schafts und einen Übertragungsadapter, wie er hier beschrieben ist, der relativ zum Schaftadapter bewegbar ist.

Insbesondere ist der Übertragungsadapter innerhalb des Schaftadapters angeordnet und relativ zum Schaftadapter translatorisch und/oder rotatorisch bewegbar. Wenn der Schaftadapter und insbesondere dessen distales Ende zur Kopplung mit einem proximalen Ende eines vorbestimmten Schafts bzw. eines Schafts eines vorbestimmten Typs ausgebildet ist, kann der Übertragungsadapter im Schaftadapter zur Kopplung mit einem proximalen Ende eines Übertragungselements, das zur Verwendung mit dem vorbestimmten Schaft bzw. mit einem Schaft des vorbestimmten Typs vorgesehen und ausgebildet ist, ausgebildet sein. Der Schaftadapter kann eine Kombination eines Schafts (und damit eines Übertragungselements) und einer Handhabungseinrichtung ermöglichen, die herkömmlich nicht für eine Kombination miteinander ausgebildet sind.

Ein Schaft oder ein Schaftadapter mit einem Übertragungsadapter mit einer bewegbaren Greifbacke, wie er hier beschrieben ist, umfasst ferner insbesondere eine seitliche Öffnung, wobei die bewegbare Greifbacke zumindest teilweise in der seitlichen Öffnung angeordnet ist, wobei die bewegbare Greifbacke ausgebildet ist, um vollständig innerhalb der Kontur des Schafts bzw. des Schaftadapters zu liegen, wenn ein proximales Ende eines Übertragungselements durch die bewegbare Greifbacke formschlüssig gehalten ist, und um beim Entkoppeln des Übertragungsadapters von einem proximalen Ende eines Übertragungselements über die Kontur des Schafts bzw. des Schaftadapters hinauszuragen.

Die seitliche Öffnung ist insbesondere in einem im Wesentlichen zylindrischen (insbesondere kreiszylindrischen) Bereich des Schafts bzw. des Schaftadapters angeordnet. Die Kontur des Schafts ist insbesondere die Kontur bzw. der äußere Rand des Querschnitts des Schafts unmittelbar proximal und/oder unmittelbar distal der seitlichen Öffnung. Insbesondere ist der Schaft bzw. der Schaftadapter im Bereich der Öffnung im Wesentlichen (d.h. von der seitlichen Öffnung abgesehen) zylindrisch oder kreiszylindrisch.

Wenn der Bereich des Schafts bzw. des Schaftadapters, der die seitliche Öffnung umfasst, in einer Ausnehmung entsprechender Gestalt in einer Handhabungseinrichtung angeordnet ist, ist die bewegbare Greifbacke durch die Oberfläche der Ausnehmung in der das proximale Ende eines Übertragungselements formschlüssig haltenden Position gehalten und an einer Auslenkung aus dieser Position gehindert. Dadurch ist die mechanische Kopplung zwischen dem Übertragungsadapter und dem Übertragungselement verriegelt. Nur wenn der Schaft bzw. Schaftadapter aus der Ausnehmung in der Handhabungseinrichtung entnommen ist, können die bewegbaren Greifbacken aus ihrer das proximale Ende eines Übertragungselements formschlüssig haltenden Position über die Kontur des Schafts bzw. des Schaftadapters hinausragend ausgelenkt und so die mechanische Kopplung zwischen Übertragungsadapter und Übertragungselement hergestellt oder gelöst werden.

Ein medizinisches Instrument umfasst eine Handhabungseinrichtung mit einem ersten Teil und einem zweiten Teil, das gegenüber dem ersten Teil bewegbar ist, einen Schaft mit einem proximalen Ende, das mit dem ersten Teil der Handhabungseinrichtung mechanisch koppelbar ist, und einem distalen Ende, ein im Schaft bewegbares Übertragungselement zur Übertragung zumindest entweder einer Kraft oder eines Drehmoments zwischen dem zweiten Teil der Handhabungseinrichtung und dem distalen Ende des Schafts und einen Übertragungsadapter, wie er hier beschrieben ist.

Ein Werkzeug kann am distalen Ende des Schafts vorgesehen oder mit dem distalen Ende des Schafts mechanisch koppelbar sein. Das Übertragungselement ist im Schaft insbesondere translatorisch und/oder rotatorisch bewegbar. Das zweite Teil der Handhabungseinrichtung ist gegenüber dem ersten Teil insbesondere schwenkbar.

Bei einem medizinischen Instrument, wie es hier beschrieben ist, ist der Übertragungsadapter insbesondere Bestandteil eines Schafts, wie er hier beschrieben ist, oder eines Schaftadapters, wie er hier beschrieben ist.

Ein medizinisches Instrument, wie es hier beschrieben ist, umfasst insbesondere eine Mehrzahl von alternativ verwendbaren Übertragungselementen mit unterschiedlich ausgebildeten proximalen Enden und eine Mehrzahl von alternativ verwendbaren Übertragungsadaptern, wobei die ersten Kupplungen aller Übertragungsadapter gleich und zur lösbaren mechanischen Kopplung mit dem zweiten Teil der Handhabungseinrichtung ausgebildet sind, wobei die zweiten Kupplungen der Mehrzahl von Übertragungsadaptern unterschiedlich ausgebildet sind, und wobei jeder Übertragungsadapter eine zweite Kupplung aufweist, die zur lösbaren mechanischen Kopplung mit einem proximalen Ende von einem der Übertragungselemente ausgebildet ist.

Insbesondere existiert zu jedem Übertragungselement ein Übertragungsadapter, dessen zweite Kupplung zur lösbaren mechanischen Kopplung mit dem proximalen Ende des Übertragungselements ausgebildet ist.

### Kurzbeschreibung der Figuren

Nachfolgend werden Ausführungsformen anhand der beigefügten Figuren näher erläutert. Es zeigen:
- Figur 1: eine schematische Darstellung eines medizinischen Instruments;
- Figur 2: eine schematische Darstellung eines Übertragungsadapters in einem Schaft;
- Figur 3: eine weitere schematische Darstellung des Übertragungsadapters aus Figur 2;
- Figur 4: eine schematische Darstellung eines Übertragungsadapters in einem Schaftadapter;
- Figur 5: eine schematische Darstellung eines Übertragungsadapters;
- Figur 6: eine weitere schematische Darstellung des Übertragungsadapters aus Figur 5;
- Figur 7: eine weitere schematische Darstellung des Übertragungsadapters aus den Figuren 5 und 6;
- Figur 8: eine weitere schematische Darstellung des Übertragungsadapters aus den Figuren 5 bis 7;
- Figur 9: eine schematische Darstellung eines weiteren Übertragungsadapters;
- Figur 10: eine schematische Darstellung eines weiteren Übertragungsadapters;
- Figur 11: eine schematische Darstellung eines weiteren Übertragungsadapters;
- Figur 12: eine schematische Darstellung eines weiteren Übertragungsadapters;
- Figur 13: eine schematische Darstellung eines weiteren Übertragungsadapters;
- Figur 14: eine weitere schematische Darstellung des Übertragungsadapters aus Figur 13;
- Figur 15: eine weitere schematische Darstellung des Übertragungsadapters aus den Figuren 13 und 14;
- Figur 16: eine schematische Darstellung eines weiteren Übertragungsadapters;
- Figur 17: schematische Darstellungen eines weiteren Übertragungsadapters;
- Figur 18: eine schematische Darstellung eines weiteren Übertragungsadapters;
- Figur 19: eine weitere schematische Darstellung des Übertragungsadapters aus Figur 18.

### Beschreibung der Ausführungsformen

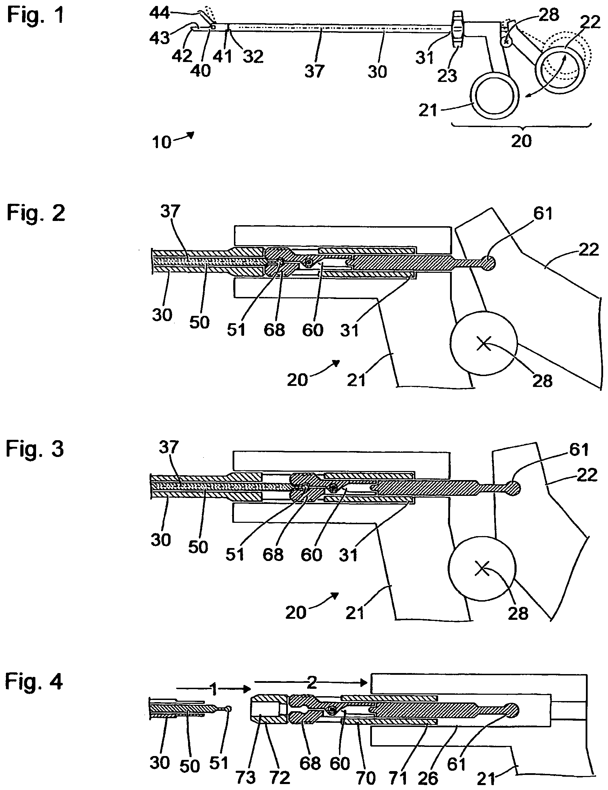

Figur 1 zeigt eine schematische Darstellung eines medizinischen Instruments 10, insbesondere eines mikroinvasiv-chirurgischen Instruments, mit einer Handhabungseinrichtung 20 am proximalen Ende, einem Schaft 30 und einem Werkzeug 40.

Die Handhabungseinrichtung umfasst ein erstes bzw. feststehendes Teil 21, ein zweites bzw. bewegbares Teil 22 und ein Rad 23. Das zweite Teil 22 ist mit dem ersten Teil 21 durch ein Gelenk derart verbunden, dass es relativ zum ersten Teil 21 um eine Schwenkachse 28 senkrecht zur Zeichenebene der Figur 1 schwenkbar ist.

Der Schaft 30 umfasst ein proximales Ende 31, das mit der Handhabungseinrichtung 20 lösbar mechanisch gekoppelt ist, und ein distales Ende 32, das mit dem Werkzeug 40 lösbar mechanisch gekoppelt ist. Der Schaft 30 ist starr oder flexibel, gerade oder gekrümmt. Bei dem dargestellten Beispiel ist der Schaft 30 gerade und weist eine Längsachse 37 auf, zu der der Schaft 30 rotationssymmetrisch ist. Das proximale Ende 31 des Schafts 30 ist mit dem Rad 23 so mechanisch gekoppelt, dass mittels des Rads 23 der Schaft 30 und das Werkzeug 40 am distalen Ende 32 des Schafts 30 um die Längsachse 37 des Schafts 30 gedreht werden können.

Das Werkzeug 40 weist ein proximales Ende 41, das mit dem distalen Ende 32 des Schafts 30 lösbar mechanisch gekoppelt ist, und ein distales Ende 42 auf. Am distalen Ende 42 weist das Werkzeug 40 ein feststehendes Maulteil 43 und ein schwenkbares Maulteil 44 auf. Das schwenkbare Maulteil 44 ist über eine in Figur 1 nicht dargestellte und im Schaft 30 bewegbare Übertragungsstange mit dem zweiten Teil 22 der Handhabungseinrichtung 20 gekoppelt, so dass eine Bewegung des zweiten Teils 22 der Handhabungseinrichtung 20 relativ zum ersten Teil 21 eine Bewegung des schwenkbaren Maulteils 44 relativ zum feststehenden Maulteil 43 um eine Schwenkachse senkrecht zur Zeichenebene der Figur 1 bewirkt. Dazu ist die in Figur 1 nicht dargestellte Übertragungsstange insbesondere innerhalb des Schafts 30 parallel zur Längsachse 37 des Schafts 30 translatorisch bewegbar, um eine Zug- oder Druckkraft zwischen dem zweiten Teil 22 der Handhabungseinrichtung 20 und des schwenkbaren Maulteils 44 des Werkzeugs 40 zu übertragen. Alternativ oder zusätzlich kann die Übertragungsstange im Schaft 30 (insbesondere um die Längsachse 37 des Schafts 30) rotierbar sein, um ein Drehmoment zwischen der Handhabungseinrichtung 20 und dem Werkzeug 40 zu übertragen.

In Figur 1 sind in durchgezogenen Linien das schwenkbare Maulteil 44 des Werkzeugs 40 in einer offenen Position und das zweite Teil 22 der Handhabungseinrichtung 20 in einer korrespondierenden Position dargestellt. In gestrichelten Linien sind das schwenkbare Maulteil 44 des Werkzeugs 40 in einer überoffenen Position und das zweite Teil 22 der Handhabungseinrichtung 20 in einer korrespondierenden Position dargestellt. Wenn das zweite Teil 22 der Handhabungseinrichtung 20 ausgehend von der in durchgezogenen Linien dargestellten Position in Richtung zum ersten Teil 21 geschwenkt wird, wird das schwenkbare Maulteil 44 des Werkzeugs 40 ausgehend von der in durchgezogener Linie dargestellten offenen Position zum feststehenden Maulteil 43 hin geschwenkt.

Figur 2 zeigt eine schematische und vergrößerte Darstellung eines Teilbereichs des medizinischen Instruments 10 aus Figur 1. Insbesondere ist ein Schnitt durch das proximale Ende 31 des Schafts 30 und einen Teil der Handhabungseinrichtung 20 gezeigt. Die Schnittebene enthält die Längsachse 37 des Schafts 30. Schnittflächen des Schafts 30, einer Übertragungs- bzw. Zugstange bzw. eines Übertragungselements 50 und eines Übertragungsadapters 60 sind schraffiert dargestellt. Die Teile 21, 22 der Handhabungseinrichtung sind lediglich in Umrissen skizziert, um sie klarer von Schaft 30, Übertragungselement 50 und Übertragungsadapter 60 abzuheben. Das in Figur 1 angedeutet Rad 23 sowie Einrichtungen zur rotatorischen Kopplung des Rads 23 mit dem Schaft 30 oder mit dem Übertragungselement 50 sind in Figur 2 nicht gezeigt.

Das proximale Ende 31 des Schafts 30 ist in einer Ausnehmung korrespondierender Gestalt im ersten Teil 21 der Handhabungseinrichtung 20 angeordnet. Der Schaft 30 bzw. dessen proximales Ende 31 kann in der Handhabungseinrichtung 20 bzw. dessen ersten Teil 21 lösbar mechanisch verriegelt sein, beispielsweise mittels einer in Figur 2 nicht dargestellten federbelasteten Klinke, die in eine Ausnehmung am Schaft 30 eingreift.

Einem Rohr ähnlich weist der Schaft 30 ein Lumen auf, in dem das Übertragungselement 50 angeordnet ist. Das Lumen im Schaft 30 und das Übertragungselement 50 weisen jeweils im Wesentlichen eine kreiszylindrische Gestalt auf. Der Querschnitt des Lumens im Schaft 30 und der Querschnitt des Überträgungselements 50 sind so gewählt, dass das Übertragungselement 50 im Schaft 30 in einer Richtung parallel zur Längsachse 37 des Schafts 30 verschiebbar und um die Längsachse 37 rotierbar und dabei spiel- und reibungsarm geführt ist. Ein proximales Ende 51 des Übertragungselements 50 ist im Wesentlichen kugelförmig ausgebildet.

Der Übertragungsadapter 60 ist überwiegend innerhalb des Schafts 30 nahe dessen proximalem Ende 31 angeordnet. Der Übertragungsadapter 60 umfasst eine erste Kupplung 61 an seinem proximalen Ende und eine zweite Kupplung 68 an seinem distalen Ende. Die erste Kupplung 61 weist im Wesentlichen die Gestalt einer Kugel auf und ist gelenkig mit dem zweiten Teil 22 der Handhabungseinrichtung 20 gekoppelt. In der in Figur 2 dargestellten Position des zweiten Teils 22 der Handhabungseinrichtung 20, die zu der oben anhand der Figur 1 dargestellten überoffenen Position des schwenkbaren Maulteils 44 des Werkzeugs 40 korrespondiert, kann die mechanische Kopplung zwischen der ersten Kupplung 61 des Übertragungsadapters 60 und dem zweiten Teil 22 der Handhabungseinrichtung 20 hergestellt oder gelöst werden. Die mechanische Kopplung zwischen der ersten Kupplung 61 des Übertragungsadapters 60 und dem zweiten Teil 22 der Handhabungseinrichtung 20 entspricht insbesondere einer herkömmlichen mechanischen Kopplung zwischen dem proximalen Ende eines herkömmlichen Übertragungselements und dem schwenkbaren Teil einer herkömmlichen Handhabungseinrichtung. Deshalb sind Details dieser mechanischen Kopplung hier nicht dargestellt.

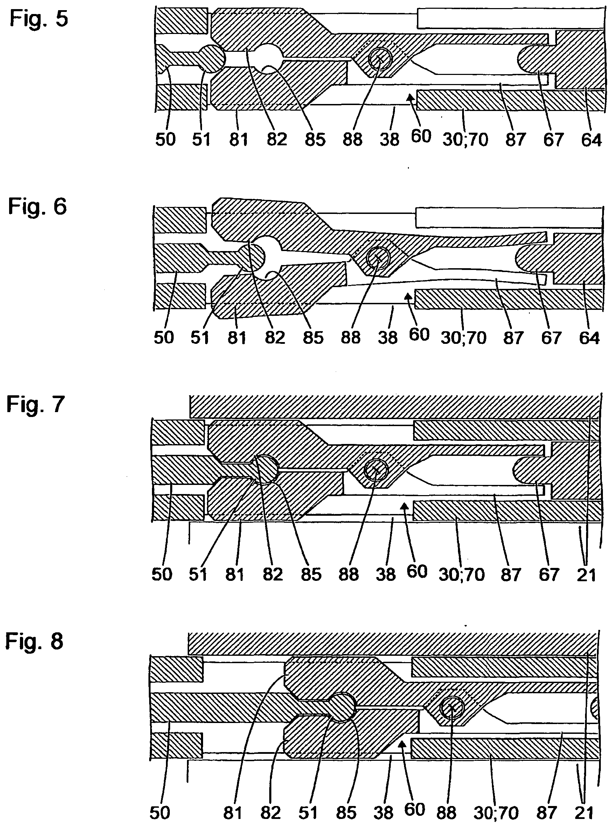

Die zweite Kupplung 68 am distalen Ende des Übertragungsadapters 60 ist mit dem proximalen Ende 51 des Übertragungselements 50 mechanisch gekoppelt. Details sind unten mit Bezug auf die Figuren 5 bis 8 dargestellt.

Das Übertragungselement 50 und der Übertragungsadapter 60 sind parallel zur Längsachse 37 des Schafts 30 verschiebbar. Figur 3 zeigt eine weitere schematische Darstellung, die hinsichtlich der dargestellten Merkmale und der Schnittebene der Figur 2 entspricht. Gegenüber der Darstellung in Figur 2 sind das Übertragungselement 50 und der Übertragungsadapter 60 in der Darstellung in Figur 3 jedoch nach proximal verschoben. Entsprechend ist das zweite Teil 22 der Handhabungseinrichtung 20 um die Schwenkachse 28 (im Uhrzeigersinn) geschwenkt.

Zwischen dem proximalen Ende 51 des Übertragungselements 50 und der zweiten Kupplung 68 des Übertragungsadapters 60 besteht eine zwar lösbare, aber im ungelösten Zustand starre (insbesondere spielfreie oder spielarme) mechanische Verbindung. Deshalb wird jede Bewegung der ersten Kupplung 61 und jede Kraft auf die erste Kupplung 61 des Übertragungsadapters 60 in Richtung parallel zur Längsachse 37 des Schafts 30 auf das Übertragungselement 50 und von diesem zum distalen Ende des medizinischen Instruments 10 und zum Werkzeug 40 übertragen und umgekehrt.

Figur 4 zeigt eine schematische Darstellung eines Schnitts durch einen Schaft 30, ein Übertragungselement 50, einen Schaftadapter 70 und einen im Schaftadapter 70 angeordneten Übertragungsadapter 60. Die Art der Darstellung, insbesondere die dargestellte Schnittebene, entspricht im Wesentlichen der der Figuren 2 und 3. Aus Platzgründen ist lediglich ein Teilbereich des ersten Teils 21 der Handhabungseinrichtung dargestellt.

Das Ausführungsbeispiel der Figur 4 unterscheidet sich von dem Ausführungsbeispiel der Figuren 2 und 3 dadurch, dass der Übertragungsadapter 60 nicht im proximalen Ende 31 bzw. in einem Bereich nahe dem proximalen Ende 31 des Schafts 30, sondern in einem separaten Schaftadapter 70 angeordnet ist. Der Schaftadapter 70 weist ein proximales Ende 71 und ein distales Ende 72 mit einer Ausnehmung 73 auf. Der Schaftadapter 70 ist (ähnlich wie bei dem Ausführungsbeispiel der Figuren 2 und 3 ein proximaler Bereich des Schafts 30) zur Aufnahme in einer Ausnehmung 26 im ersten Teil 21 der Handhabungseinrichtung ausgebildet. Die Ausnehmung 73 am distalen Ende 72 des Schaftadapters 70 ist zur Aufnahme des proximalen Endes des Schafts 30 ausgebildet.

In Figur 4 sind der Schaft 30 mit dem Übertragungselement 50 einerseits und der Schaftadapter 70 mit dem Übertragungsadapter 60 andererseits sowie ferner das erste Teil 21 der Handhabungseinrichtung jeweils separat dargestellt. Ein mit einer "1" gekennzeichneter Pfeil deutet an, wie der Schaft 30 mit dem Übertragungselement 50 in die Ausnehmung 73 am distalen Ende 72 des Schaftadapters 70 und die zweite Kupplung 68 des Übertragungsadapters 60 eingesetzt werden kann. Ein mit einer "2" gekennzeichneter Pfeil deutet an, wie der Schaftadapter 70 mit dem Übertragungsadapter 60, die bei der Darstellung in Figur 4 erst teilweise in die Ausnehmung 26 im ersten Teil 21 der Handhabungseinrichtung eingeführt dargestellt sind, vollständig in diese eingesetzt werden können. Im Folgenden wird dargestellt, dass insbesondere die Kopplung des proximalen Endes 51 des Übertragungselements 50 mit der zweiten Kupplung 68 des Übertragungsadapters 60 entsprechend dem Pfeil "1" vor dem vollständigen Einführen des Schaftadapters 70 mit dem Übertragungsadapter 60 in die Ausnehmung 26 im ersten Teil 21 der Handhabungseinrichtung erfolgen muss.

Die Figuren 5 bis 8 zeigen schematische vergrößerte Schnittdarstellungen eines Übertragungsadapters 60, der in vielen Merkmalen den oben anhand der Figuren 2 bis 4 dargestellten Übertragungsadaptern ähnelt. Die Schnittebenen der Figuren 5 bis 8 entsprechen den Schnittebenen der Figuren 2 bis 4, sind parallel zur Längsachse 37 (vgl. Figuren 1 bis 3) des Schafts 30. Die Darstellung in den Figuren 5 bis 8 unterscheidet sich durch unterschiedliche Positionen und Zustände eines Übertragungselements 50 und des Übertragungsadapters 60.

In den Figuren 5 bis 8 ist der Übertragungsadapter 60 jeweils in einem proximalen Bereich eines Schafts 30 oder in einem Schaftadapter 70 dargestellt. Da sich ein Schaft 30 und ein Schaftadapter 70 nicht in den in den Figuren 5 bis 8 dargestellten Merkmalen, sondern nur am distalen Ende (vgl. Figuren 3 und 4) unterscheiden, gilt die nachfolgende Darstellung anhand der Figuren 5 bis 8 sowohl für einen Schaft als auch für einen Schaftadapter.

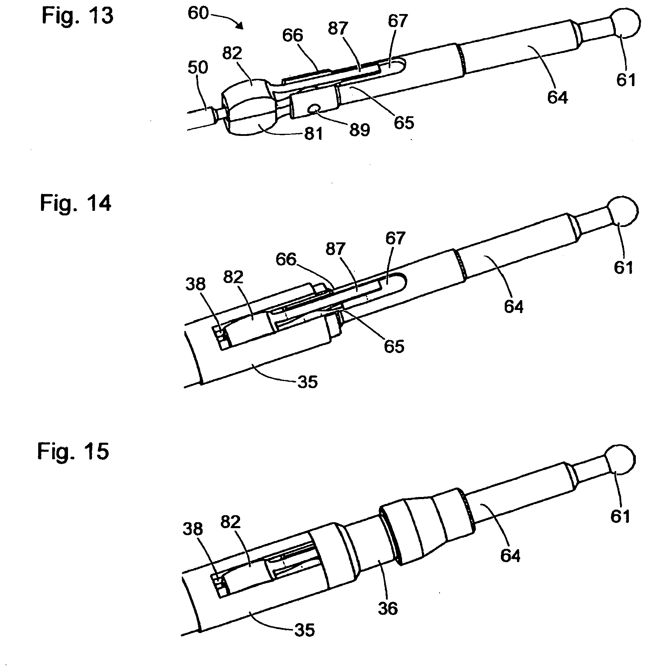

Der Übertragungsadapter 60 umfasst einen Grundkörper 64 mit einem Wiederlager 67, eine erste Greifbacke 81 und eine zweite Greifbacke 82. Die erste Greifbacke 81 und die zweite Greifbacke 82 bilden die zweite Kupplung 68 (vgl. Figuren 2 bis 4) des Übertragungsadapters 60. Die erste Greifbacke 81 und die zweite Greifbacke 82 sind gelenkig mit dem Grundkörper 64 des Übertragungsadapters 60 verbunden und unabhängig voneinander um eine Schwenkachse 88 senkrecht zu den Zeichenebenen der Figuren 5 bis 8 schwenkbar. Details der Verbindung zwischen Grundkörper 64 und Greifbacken 81, 82 über in den Figuren 5 bis 8 nicht dargestellte Holme sind unten mit Bezug auf Figur 13 beschrieben.

Der Schaft 30 bzw. der Schaftadapter 70 weisen zwei gegenüberliegende seitliche Öffnungen 38 auf. Die Greifbacken 81, 82 sind teilweise in den Öffnungen 38 angeordnet und liegen bei den in der Figur 5 gezeigten Positionen vollständig innerhalb der Kontur des Schafts 30 bzw. des Schaftadapter 70. Jede Greifbacke 81, 82 ist einstückig mit einem zugeordneten elastischen Element 87 ausgebildet. Die elastischen Elemente 87 sind jeweils balkenförmig. Von den Greifbacken 81, 82 abgewandte Enden der elastischen Elemente liegen an dem Wiederlager 67 am Grundkörper 64 des Übertragungsadapters 60 an bzw. sind an diesem abgestützt. Durch die elastischen Elemente 87 auf die Greifbacken 81, 82 ausgeübte Kräfte bzw. Drehmomente halten diese in den in Figur 5 gezeigten Positionen, solange keine äußere Kraft auf sie wirkt.

Wenn das proximale Ende 51 des Übertragungselements 50 nach proximal geschoben und dabei gegen die Greifbacken 81, 82 (insbesondere die angedeuteten schrägen Gleitflächen) gedrückt wird, werden die Greifbacken 81, 82 aus den in Figur 5 gezeigten Positionen in die in Figur 6 gezeigten Positionen ausgelenkt. Dabei werden die Greifbacken 81, 82 in entgegengesetzten Richtungen um die Schwenkachse 88 geschwenkt und die elastischen Elemente 87 elastisch verformt. Die elastische Verformung der elastischen Elemente 87 bewirkt eine rückstellende Kraft bzw. ein rückstellendes Drehmoment hin zu den in Figur 5 gezeigten Positionen der Greifbacken 81, 82.

Bei der in Figur 6 dargestellten Auslenkung der Greifbacken 81, 82 ragen die äußeren Bereiche (in Figur 6 der untere Bereich der ersten Greifbacke 81 und der obere Bereich der zweiten Greifbacke 82) über die Kontur des Schafts 30 bzw. des Schaftadapters 70 hinaus.

Wenn das Übertragungselement 50 ausgehend von der in Figur 6 gezeigten Position weiter nach proximal geschoben wird, erreicht das proximale Ende 51 des Übertragungselements 50 Ausnehmungen 85 in den Greifbacken 81, 82. Die Gestalt der Ausnehmungen 85 entspricht der Gestalt des proximalen Endes 51 des Übertragungselements 50. Insbesondere sind sowohl das proximale Ende 51 des Übertragungselements 50 als auch die Ausnehmungen 85 kugelförmig. Wenn das proximale Ende 51 des Übertragungselements 50 seine relativ zum Übertragungsadapter 60 äußerst proximale Position erreicht hat, werden die Greifbacken 81, 82 durch die elastischen Elemente 87 in die in Figur 7 gezeigten Positionen bewegt, die vollständig oder im Wesentlichen den in Figur 5 gezeigten Positionen entsprechen. Das proximale Ende 51 des Übertragungselements 50 liegt in den Ausnehmungen 85 in den Greifbacken 81, 82 und wird so von diesen formschlüssig gehalten.

Durch eine ausreichende Zugkraft auf das Übertragungselement 50 nach distal kann das proximale Ende 51 des Übertragungselements 50 aus der formschlüssigen Verbindung mit den Greifbacken 81, 82 gelöst werden. Dabei werden die Greifbacken 81, 82 gegen die durch die elastischen Elemente 87 ausgeübten Kräfte bzw. Momente (vorübergehend) in die in Figur 6 gezeigten Positionen gebracht.

In den Darstellungen der Figuren 7 und 8 sind der dargestellte proximale Bereich des Schafts 30 bzw. der Schaftadapter 70 und der Übertragungsadapter 60 in einer Ausnehmung in einem Teil 21 einer Handhabungseinrichtung angeordnet (vgl. Figuren 2 bis 4). In den Figuren 7 und 8 sind dabei jeweils nur die unmittelbar angrenzenden Wände bzw. Bereiche des Teils 21 der Handhabungseinrichtung angedeutet. Die Greifbacken 81, 82 liegen an dem Teil 21 der Handhabungseinrichtung an und sind durch diese an einer Auslenkung in die in Figur 6 dargestellten Positionen gehindert. Damit ist die mechanische Kopplung zwischen dem proximalen Ende 51 des Übertragungselements 50 und der durch die Greifbacken 81, 82 gebildeten Kupplung am distalen Ende des Übertragungsadapters 60 verriegelt. Die mechanische Verbindung zwischen dem proximalen Ende 51 des Übertragungselements 50 und dem Übertragungsadapter 60 kann deshalb nur hergestellt oder getrennt werden, wenn der Übertragungsadapter 60 sich zusammen mit dem Schaft 30 bzw. dem Schaftadapter 70 außerhalb des Teils 21 der Handhabungseinrichtung befindet.

Die seitlichen Öffnungen 38 im proximalen Bereich des Schafts 30 bzw. im Schaftadapter 70 sind wesentlich länger bzw. weisen eine in Richtung parallel zur Längsachse 37 des Schafts 30 (vgl. Figuren 1 bis 3) gemessene Länge auf, die wesentlich größer ist als die in den seitlichen Öffnungen 38 angeordneten Bereiche der Greifbacken 81, 82. Deshalb sind die Greifbacken 81, 82 in Richtung parallel zur Längsachse des Schafts bewegbar. In den Figuren 7 und 8 sind das Übertragungselement 50 und der Übertragungsadapter 60 in unterschiedlichen Positionen dargestellt, die insbesondere unterschiedlichen Positionen des schwenkbaren Maulteils 44 (vgl. Figur 1) eines Werkzeugs am distalen Ende des medizinischen Instruments entsprechen.

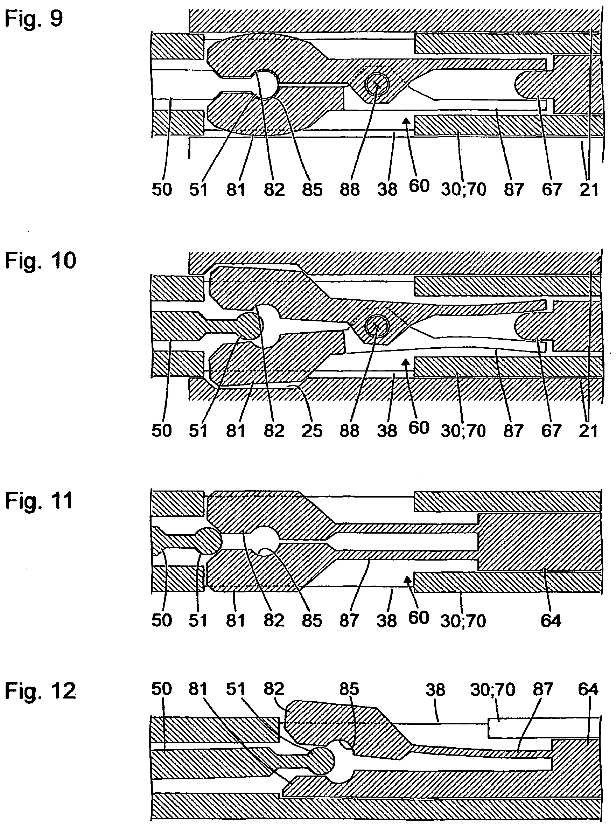

Figur 9 zeigt eine schematische Darstellung eines weiteren Übertragungsadapters 60, der in einigen Merkmalen dem oben anhand der Figuren 5 bis 8 dargestellten Übertragungsadapter ähnelt. Die Art der Darstellung, insbesondere die dargestellte Schnittebene entspricht den Darstellungen der Figuren 5 bis 8.

Der in Figur 9 dargestellte Übertragungsadapter 60 unterscheidet sich von dem oben anhand der Figuren 5 bis 8 dargestellten Übertragungsadapter insbesondere dadurch, dass die äußeren Oberflächen der Greifbacken 81, 82 konvex gewölbt sind, insbesondere zylindrisch (mit einer Zylinderachse senkrecht zur Zeichenebene) oder sphärisch.

Figur 10 zeigt eine schematische Darstellung eines weiteren Übertragungsadapters 60, der in einigen Merkmalen den oben anhand der Figuren 5 bis 9 dargestellten Übertragungsadaptern 60 ähnelt. Die Art der Darstellung, insbesondere die dargestellte Schnittebene entspricht den Darstellungen der Figuren 5 bis 9.

Im Unterschied zu den oben anhand der Figuren 7 und 8 dargestellten Eigenschaften und Merkmalen ist der Übertragungsadapter 60 der Figur 10 in einem Teil 21 einer Handhabungseinrichtung angeordnet, der Ausnehmungen 25 aufweist. Die Ausnehmungen 25 weisen jeweils eine zu den außen liegenden Bereichen der Greifbacken 81, 82 korrespondierende Gestalt auf. Deshalb können die Greifbacken 81, 82 in ihrer in Figur 10 dargestellten äußerst distalen Position in die auch in Figur 6 dargestellten Positionen ausgelenkt werden, in denen eine mechanische Verbindung mit dem proximalen Ende 51 eines Übertragungselements 50 hergestellt oder gelöst werden kann. Wenn der Übertragungsadapter 60 mit den Greifbacken 81, 82 nach proximal verschoben wird, (vgl. Figur 8) ist hingegen die mechanische Kopplung zwischen dem Übertragungselement 50 und dem Übertragungsadapter 60 verriegelt, ähnlich wie oben anhand der Figuren 7 und 8 beschrieben.

Figur 11 zeigt eine schematische Darstellung eines weiteren Übertragungsadapters 60, der in einigen Merkmalen den oben anhand der Figuren 5 bis 10 dargestellten Übertragungsadaptern 60 ähnelt. Die Darstellung in Figur 11 entspricht, insbesondere hinsichtlich der dargestellten Schnittebene, den Darstellungen in den Figuren 5 bis 10.

Bei dem Ausführungsbeispiel der Figur 11 sind die elastischen Elemente 87 als Festkörpergelenke ausgebildet, die einstückig den Grundkörper 64 mit den Greifbacken 81, 82 verbinden. Die elastischen Elemente 87 erfüllen somit gleichzeitig zwei Funktionen, nämlich die gelenkige Verbindung mit dem Grundkörper 64 und die rückstellende Wirkung.

Figur 12 zeigt eine schematische Darstellung eines weiteren Übertragungsadapters 60, der in einigen Merkmalen den oben anhand der Figuren 5 bis 11 dargestellten Übertragungsadaptern 60 ähnelt. Die Darstellung in Figur 12 entspricht, insbesondere hinsichtlich der dargestellten Schnittebene, den Darstellungen in den Figuren 5 bis 11.

Das Ausführungsbeispiel in Figur 12 unterscheidet sich von den oben anhand der Figuren 5 bis 11 dargestellten Ausführungsbeispielen insbesondere dadurch, dass die erste Greifbacke 81 starr mit dem Grundkörper 64 des Übertragungsadapters 60 verbunden ist. Nur die zweite Greifbacke 82 ist gelenkig mit dem Grundkörper 64 verbunden, und zwar in dem dargestellten Beispiel durch ein als Festkörpergelenk ausgebildetes elastisches Element 87. Beim Herstellen oder Lösen einer mechanischen Verbindung zwischen dem Übertragungselement 50 und dem Übertragungsadapter 60 findet lediglich eine Auslenkung der zweiten Greifbacke 82 statt. Diesem asymmetrischen Vorgang entsprechend ist eine Auslenkung des Übertragungselements 50, insbesondere seines proximalen Endes 51 erforderlich, die in Figur 12 erkennbar ist.

Die Figuren 13 bis 15 zeigen schematische axonometrische Darstellungen eines Übertragungsadapters 60, der in einigen Merkmalen den oben anhand der Figuren 5 bis 10 dargestellten Übertragungsadaptern, insbesondere dem oben anhand der Figur 9 dargestellten Übertragungsadapter entspricht.

In Figur 13 ist lediglich der Übertragungsadapter 60 mit einem mechanisch gekoppelten Übertragungselement 50 dargestellt. Der Grundkörper 64 des Übertragungsadapters 60 weist an seinem proximalen Ende die kugelförmige erste Kupplung 61 und an seinem distalen Ende zwei gabelförmig angeordnete Holme 65, 66 auf. Die Greifbacken 81, 82 sind durch eine Welle 89 parallel zu ihrer Schwenkachse 88 (vgl. Figuren 5 bis 10) mit den Holmen 65, 66 verbunden. Je ein Ende der Welle 89 ist mit einem Holm 65, 66 gefügt. Die Lagerung der Greifbacken 81, 82 auf der Welle 89 ermöglicht die beschriebene Schwenkbarkeit. Die mit den Greifbacken 81, 82 einstückig ausgebildeten elastischen Elemente 87 liegen am Wiederlager 67 am Grundkörper 64 an.

In der Darstellung in Figur 14 sind das distale Ende des Übertragungsadapters und insbesondere die Greifbacken 82 in einer distalen Hülse 35 angeordnet. Bei der Darstellung in Figur 15 ist die distale Hülse 35 durch eine proximale Hülse 36 zum proximalen Bereich eines Schafts (oder eines Schaftadapters) ergänzt. Die seitlichen Öffnungen 38, in denen die Greifbacken 82 teilweise angeordnet sind, sind durch Längsschlitze in der distalen Hülse 35 gebildet.

Anders als in den Figuren 2 bis 12 angedeutet, weist der durch die Hülsen 35, 36 gebildete proximale Bereich des Schafts, insbesondere die proximale Hülse 36, keinen kreiszylindrischen Querschnitt auf. Eine breite und flache ringförmige Nut ermöglicht eine formschlüssige Verriegelung einer mechanischen Verbindung des Schafts mit einer Handhabungseinrichtung durch Einrichtungen, die in den Figuren 1 bis 12 nicht dargestellt sind.

Bei den oben anhand der Figuren 2 bis 15 dargestellten Ausführungsbeispielen umfasst die zweite Kupplung 68 jeweils zwei Greifbacken 81, 82, von denen zumindest eine schwenkbar ist. Alternativ kann die zweite Kupplung 68 eine oder mehr Greifbacken umfassen, die entlang eines vorbestimmten geraden oder gekrümmten Pfads bewegbar bzw. verschiebbar sind. Eine Greifbacke kann gleichzeitig schwenkbar und entlang eines vorbestimmten Pfads bewegbar sein. Ein Pfad ist beispielsweise durch eine Kulisse oder eine ähnliche Führung vorbestimmt, die eine Translationsbewegung mit einer Schwenkbewegung koppeln kann.

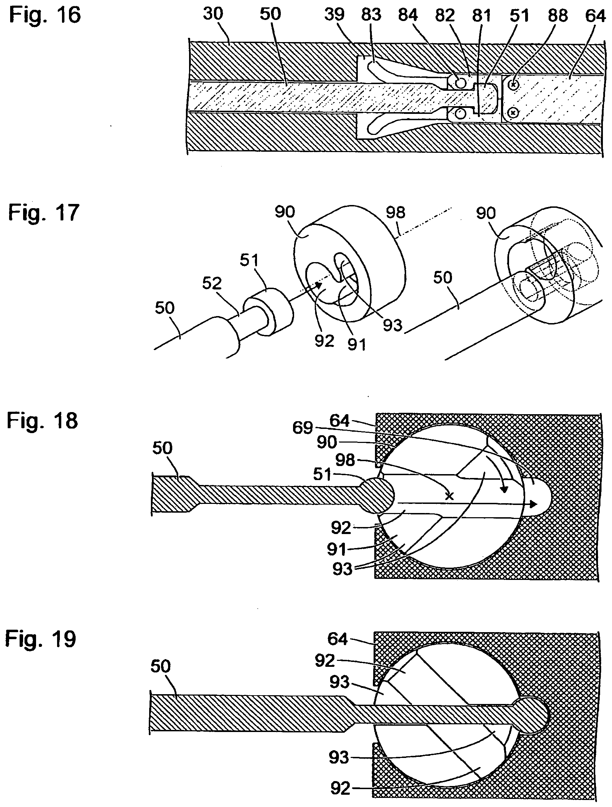

Figur 16 zeigt eine schematische Darstellung eines Schnitts durch einen weiteren Übertragungsadapter. Die Schnittebene ist parallel zur Zeichenebene der Figur 1 und entspricht im Wesentlichen den Schnittebenen der Figuren 2 bis 12. Ähnlich wie bei den Figuren 5 bis 12 ist lediglich das distale Ende des Übertragungsadapters dargestellt. Das proximale Ende des Übertragungsadapters entspricht beispielsweise dem oben anhand der Figuren 2 bis 4 dargestellten proximalen Ende.

Die in Figur 16 dargestellte zweite Kupplung des Übertragungsadapters umfasst zwei Greifbacken 81, 82. Jede Greifbacke ist um eine Schwenkachse 88 senkrecht zur Schnittebene der Figur 16 schwenkbar. Jede Schwenkachse 88 ist durch einen Stift bzw. einen Bolzen bzw. eine Welle definiert, mittels dessen die Greifbacke 81, 82 mit einem Grundkörper 64 des Übertragungsadapters gelenkig verbunden ist.

Der Übertragungsadapter ist in einem Schaft 30, insbesondere in einem proximalen Bereich des Schafts 30 angeordnet. Das Lumen des Schafts 30, in dem ein Übertragungselement 50 angeordnet ist, ist zu einem Hohlraum erweitert, der auch den Übertragungsadapter aufnimmt. Das distale Ende 39 des Hohlraums ist ausgebildet, um ein Schwenken der Greifbacken 81, 82 nach außen zu ermöglichen, wenn der Übertragungsadapter mit dem Grundkörper 64 und den Greifbacken 81, 82 gegenüber der in Figur 16 dargestellten Position nach distal verschoben ist.

Nahe dem distalen Ende 39 des Hohlraums sind Kulissenführungen 83 angeordnet. Die Kulissenführungen 83 weisen jeweils einen im Wesentlichen geraden proximalen Abschnitt und einen gekrümmten distalen Abschnitt auf. An jeder Greifbacke 81, 82 ist nahe deren distalem Ende ein Stift 84 angeordnet, der in eine der Kulissenführungen 83 eingreift. Durch die Kulissenführungen 83 und die in die Kulissenführungen 83 eingreifenden Stifte 84 ist eine lineare Bewegung des Übertragungsadapters zwischen einer in Figur 16 dargestellten proximalen Position und einer distalen Position mit einer Schwenkbewegung der Greifbacken 81, 82 um deren Schwenkachsen 88 gekoppelt. Bei der in Figur 16 gezeigten proximalen Position des Übertragungsadapters und in einem Bereich distal dieser Position befinden sich die Greifbacken 81, 82 in den in Figur 16 gezeigten greifenden bzw. haltenden Positionen, in denen Sie das proximale Ende 51 des Übertragungselements 50 formschlüssig halten. In einem äußerst proximalen Bereich schwenken die Greifbacken 81, 82 auseinander und geben das proximale Ende 51 des Übertragungselements 50 frei.

Figur 17 zeigt zwei schematische axonometrische Darstellungen eines weiteren Übertragungsadapters. Insbesondere ist ein Kupplungsglied 90 des Übertragungsadapters dargestellt, das mit einem proximalen Ende 51 eines Übertragungselements gekoppelt werden kann. Weitere Elemente und Bauteile des Übertragungsadapters und ein Schaft oder Schaftadapter, in dem der Übertragungsadapter angeordnet sein kann, sind nicht dargestellt.

Das Kupplungsglied 90 ist um eine Rotationsachse 98 rotierbar, die im Wesentlichen parallel zur Längsachse eines mit dem Kupplungsglied 90 zu koppelnden Übertragungselements 50 ist. Das Kupplungsglied 90 weist eine Öffnung bzw. Ausnehmung 91 mit einem ersten Ende 92 und einem zweiten Ende 93 auf. Das erste Ende 92 weist einen Querschnitt (bezogen auf eine Ebene senkrecht zur Rotationsachse 98) auf, der an das proximale Ende 51 des Übertragungselements 50 angepasst ist. Das proximale Ende 51 des Übertragungselements 50 kann deshalb durch das erste Ende 92 der Ausnehmung 91 hindurchgeführt werden, wenn das erste Ende 92 der Ausnehmung 91 zum proximalen Ende 51 des Übertragungselements 50 so ausgerichtet ist, wie in Figur 17 links dargestellt.

Das zweite Ende 92 der Ausnehmung 91 weist einen Querschnitt auf, der an den Querschnitt eines Halses 52 distal des proximalen Endes 51 des Übertragungselements 50 angepasst ist, Deshalb kann das rotierbare Kupplungsglied 90 um seine Rotationsachse 98 in die in Figur 17 rechts dargestellte Position rotiert werden, wenn der Hals 52 des Übertragungselements in der Ausnehmung 91 liegt. In der in Figur 17 rechts dargestellten Position des rotierbaren Kupplungsglieds 90 ist das Übertragungselement 50 formschlüssig mit dem Kupplungsglied 90 gekoppelt.

Die Figuren 18 und 19 zeigen schematische Schnittdarstellungen eines weiteren Übertragungsadapters. Insbesondere ist ein distales Ende des Übertragungsadapters mit einer Kupplung zum Koppeln mit einem proximalen Ende 51 eines Übertragungselements 50 gezeigt. Ein in den Figuren 18 und 19 nicht dargestelltes proximales Ende des Übertragungsadapters entspricht beispielsweise dem oben anhand der Figuren 2 bis 4 dargestellten proximalen Ende. Die Schnittebene ist parallel zur Zeichenebene der Figur 1 und entspricht im Wesentlichen den Schnittebenen der Figuren 2 bis 12 und 16.

Der Übertragungsadapter umfasst in einem Grundkörper 64 ein Kupplungsglied 90, das um eine Rotationsachse 98 senkrecht zur Schnittebene der Figuren 18 und 19 rotierbar ist. Das Kupplungsglied 90 ist in den Figuren 18 und 19 in zwei verschiedenen Positionen gezeigt. Das Kupplungsglied 90 weist einen im Wesentlichen kreisförmigen Querschnitt auf und ist in einem entsprechenden Hohlraum im Grundkörper 64 angeordnet. Das Kupplungsglied 90 weist eine Ausnehmung 91 auf, die das Kupplungsglied 90 parallel zur Schnittebene der Figuren 18 und 19 von distal nach proximal vollständig durchdringt.

Die Ausnehmung umfasst eine im Wesentlichen kreiszylindrische Durchgangsbohrung 92, die an jedem Enden jeweils eine schlüssellochartige Erweiterung 93 in Richtung gegen den Uhrzeigersinn und parallel zur Schnittebene der Figuren 18 und 19 aufweist. Hinsichtlich ihrer mechanischen Wechselwirkung mit dem Übertragungselement 50 bzw. dessen proximalem Ende 51 entsprechen die kreiszylindrische Durchgangsbohrung 92 dem ersten Ende und die schlüssellochartigen Erweiterungen 93 dem zweiten Ende der Ausnehmung im rotierbaren Kupplungsglied des Ausführungsbeispiels aus Figur 17.

Bei der in Figur 18 gezeigten Position des Kupplungsglieds 90 ist die Durchgangsbohrung 92 parallel zur Längsachse des Übertragungselements 50. Das proximale Ende 51 des Übertragungselements 50 kann, wie durch einen geraden Pfeil angedeutet, durch die Durchgangsbohrung 92 hindurch bis in eine Nische 69 im Grundkörper 64 geführt werden. Danach kann das Kupplungsglied 90, wie durch einen gekrümmten Pfeil angedeutet, um seine Rotationsachse 98 bis zu der in Figur 19 gezeigten Position rotiert werden. In der in Figur 19 gezeigten Position des Kupplungsglieds 90 ist das proximale Ende 51 des Übertragungselements durch das Kupplungsglied 90 formschlüssig in der Nische 69 gehalten.

### Bezugszeichen

- 10: medizinisches Instrument
- 20: Handhabungseinrichtung
- 21: erstes Teil der Handhabungseinrichtung 20
- 22: zweites Teil der Handhabungseinrichtung 20
- 23: Rad an der Handhabungseinrichtung 20
- 25: Ausnehmung
- 26: Ausnehmung im zweiten Teil 22
- 28: Schwenkachse des zweiten Teils 22
- 30: Schaft
- 31: proximales Ende des Schafts 30
- 32: distales Ende des Schafts 30
- 35: distale Hülse
- 36: proximale Hülse
- 37: Längsachse des Schafts 30
- 38: seitliche Öffnung am proximalen Ende 31 des Schafts 30 oder des Schaftadapters 70
- 39: distales Ende eines Hohlraums
- 40: Werkzeug
- 41: proximales Ende des Werkzeugs 40
- 42: distales Ende des Werkzeugs 40
- 43: feststehendes Maulteil des Werkzeugs 40
- 44: schwenkbares Maulteil des Werkzeugs 40
- 50: Übertragungselement
- 51: proximales Ende des Übertragungselements 50
- 52: Hals distal des proximalen Endes 51 des Übertragungselements 50
- 60: Übertragungsadapter
- 61: erste Kupplung des Übertragungsadapters 60
- 64: Grundkörper des Übertragungsadapters 60
- 65: erster Holm
- 66: zweiter Holm
- 67: Widerlager für elastische Abschnitte 87 der Greifbacken 81, 82
- 68: zweite Kupplung des Übertragungsadapters 60
- 69: Nische im Grundkörper 64
- 70: Schaftadapter
- 71: proximales Ende des Schaftadapters 70
- 72: distales Ende des Schaftadapters 70
- 73: Ausnehmung am distalen Ende 72 des Schaftadapters 70
- 81: erste Greifbacke der zweiten Kupplung 68
- 82: zweite Greifbacke der zweiten Kupplung 68
- 83: Kulissenführung
- 84: Stift
- 85: konkaver Abschnitt an der ersten Greifbacke 81
- 87: elastisches Element
- 88: Schwenkachse der Greifbacken 81, 82
- 89: Welle
- 90: rotierbares Kupplungsglied
- 91: Ausnehmung im rotierbaren Kupplungsglied 90
- 92: erstes Ende der Ausnehmung 91
- 93: zweites Ende der Ausnehmung 91
- 98: Rotationsachse des rotierbaren Kupplungsglieds 90

## Patentansprüche

1. Übertragungsadapter (60) für ein medizinisches Instrument (10) mit einem Schaft (30), einem im Schaft (30) bewegbaren Übertragungselement (50) und einer Handhabungseinrichtung (20) mit einem ersten Teil (21), das mit einem proximalen Ende (31) des Schafts (30) mechanisch starr koppelbar ist, und einem zweiten Teil (22), das gegenüber dem ersten Teil (21) bewegbar ist, wobei der Übertragungsadapter (60) aufweist:
eine erste Kupplung (61) zur lösbaren mechanischen Kopplung mit dem zweiten Teil (22) der Handhabungseinrichtung (20);
eine zweite Kupplung (68) zur lösbaren formschlüssigen mechanischen Kopplung mit einem proximalen Ende (51) des Übertragungselements (50),
**dadurch gekennzeichnet, dass** der Übertragungsadapter (60) in einen proximalen Bereich des Schafts (30) derart integriert ist, dass er nicht zerstörungsfrei oder nicht ohne Verwendung von Werkzeug aus dem Schaft entnommen werden kann.

2. Übertragungsadapter (60) nach dem vorangehenden Anspruch, bei dem die zweite Kupplung (68) eine bewegbare Greifbacke (81, 82) zum form- oder kraftschlüssigen Halten des proximalen Endes (51) des Übertragungselements (50) umfasst.

3. Übertragungsadapter (60) nach dem vorangehenden Anspruch, bei dem die bewegbare Greifbacke (81, 82) um eine Schwenkachse (88) schwenkbar ist.

4. Übertragungsadapter (60) nach Anspruch 2, bei dem die bewegbare Greifbacke (81, 82) entlang eines vorbestimmten Pfads bewegbar ist.

5. Übertragungsadapter (60) nach einem der Ansprüche 2 und 3, ferner mit:
einem elastischen Element (87), das vorgesehen und ausgebildet ist, um die bewegbare Greifbacke (81, 82) zu einer das proximale Ende (51) des Übertragungselements (50) greifenden Position hin mechanisch vorzuspannen.

6. Übertragungsadapter (60) nach einem der vorangehenden Ansprüche, bei dem die zweite Kupplung (68) ausgebildet ist, um verriegelt zu sein, wenn der Übertragungsadapter (60) in einer Handhabungseinrichtung (20) angeordnet ist.

7. Übertragungsadapter (60) nach einem der vorangehenden Ansprüche, bei dem die erste Kupplung (61) des Übertragungsadapters (60) einen kugelflächenförmigen Oberflächenabschnitt aufweist.

8. Schaft (30) für ein medizinisches Instrument (10), mit:
einem proximalen Ende (31) zur Kopplung mit einem ersten Teil (21) einer Handhabungseinrichtung (20);
einem distalen Ende (32) zur Kopplung mit einem Werkzeug (40);
einem Übertragungsadapter (60) nach einem der vorangehenden Ansprüche, der relativ zum Schaft (30) bewegbar ist.

9. Schaft (30) nach Anspruch 8 in Rückbezug auf einen der Ansprüche 2 bis 5, ferner mit:
einer seitlichen Öffnung (38) im Schaft (30), wobei die bewegbare Greifbacke (81, 82) zumindest teilweise in der seitlichen Öffnung (38) angeordnet ist,
wobei die bewegbare Greifbacke (81, 82) ausgebildet ist, um vollständig innerhalb der Kontur des Schafts (30) zu liegen, wenn ein proximales Ende (51) eines Übertragungselements (50) durch die bewegbare Greifbacke (81, 82) formschlüssig gehalten ist, und um beim Entkoppeln des Übertragungsadapters (60) von einem proximalen Ende (51) eines Übertragungselements (50) über die Kontur des Schafts (30) hinauszuragen.

10. Medizinisches Instrument (10) mit:
einer Handhabungseinrichtung (20) mit einem ersten Teil (21) und einem zweiten Teil (22), das gegenüber dem ersten Teil (21) bewegbar ist;
einem Schaft (30) mit einem proximalen Ende (31), das mit dem ersten Teil (21) der Handhabungseinrichtung (20) mechanisch koppelbar ist, und einem distalen Ende (32);
einem im Schaft (30) bewegbaren Übertragungselement (50) zur Übertragung zumindest entweder einer Kraft oder eines Drehmoments zwischen dem zweiten Teil (21) der Handhabungseinrichtung (20) und dem distalen Ende (32 des Schafts (30);
einem Übertragungsadapter (60) nach einem der Ansprüche 1 bis 7.

11. Medizinisches Instrument (10) nach dem vorangehenden Anspruch, bei dem der Übertragungsadapter (60) Bestandteil eines Schafts (30) nach Anspruch 8 oder 9.

12. Medizinisches Instrument (10) nach einem der Ansprüche 10 und 11, mit:
einer Mehrzahl von alternativ verwendbaren Übertragungselementen (50) mit unterschiedlich ausgebildeten proximalen Enden (51);
einer Mehrzahl von alternativ verwendbaren Übertragungsadaptern (60),
wobei die ersten Kupplungen (61) aller Übertragungsadapter (60) gleich und zur lösbaren mechanischen Kopplung mit dem zweiten Teil (22) der Handhabungseinrichtung (20) ausgebildet sind,
wobei die zweiten Kupplungen (68) der Mehrzahl von Übertragungsadaptern (60) unterschiedlich ausgebildet sind,
wobei jeder Übertragungsadapter (60) eine zweite Kupplung (68) aufweist, die zur lösbaren mechanischen Kopplung mit einem proximalen Ende (51) von einem der Übertragungselemente (50) ausgebildet ist.

## Claims

1. Transmission adapter (60) for a medical instrument (10) with a shaft (30), a transmission element (50) movable in the shaft (30), and a handling device (20) with a first part (21), which can be coupled mechanically rigidly to a proximal end (31) of the shaft (30), and a second part (22), which is movable relative to the first part (21), wherein the transmission adapter (60) has:
a first coupling element (61) for releasable mechanical coupling to the second part (22) of the manipulating device (20);
a second coupling element (68) for releasable positive locking mechanical coupling to a proximal end (51) of the transmission element (50),
**characterized in that** the transmission adapter (60) is integrated into a proximal region of the shaft (30) such that it cannot be removed from the shaft non-destructively or without using a tool.

2. Transmission adapter (60) according to the preceding claim, wherein the second coupling element (68) comprises a movable grip jaw (81, 82) for holding the proximal end (51) of the transmission element (50) with a form fit or force fit.

3. Transmission adapter (60) according to the preceding claim, wherein the movable grip jaw (81, 82) is pivotable about a pivot axis (88).

4. Transmission adapter (60) according to Claim 2, wherein the movable grip jaw (81, 82) is movable along a predetermined path.

5. Transmission adapter (60) according to either of Claims 2 and 3, further comprising:
an elastic element (87), which is provided and designed to mechanically bias the movable grip jaw (81, 82) towards a position gripping the proximal end (51) of the transmission element (50).

6. Transmission adapter (60) according to one of the preceding claims, wherein the second coupling element (68) is designed to be locked when the transmission adapter (60) is arranged in a handling device (20).

7. Transmission adapter (60) according to one of the preceding claims, wherein the first coupling element (61) of the transmission adapter (60) has a surface portion in the shape of a spherical surface.

8. Shaft (30) for a medical instrument (10), with:
a proximal end (31) for coupling to a first part (21) of a handling device (20);
a distal end (32) for coupling to a tool (40);
a transmission adapter (60) according to one of the preceding claims, which is movable relative to the shaft (30).

9. Shaft (30) according to Claim 8 with reference to one of the Claims 2 to 5, further comprising:
a lateral opening (38) in the shaft (30), wherein the movable grip jaw (81, 82) is arranged at least partially in the lateral opening (38),
wherein the movable grip jaw (81, 82) is designed to lie completely within the contour of the shaft (30) when a proximal end (51) of a transmission element (50) is held with a form fit by the movable grip jaw (81, 82), and to protrude beyond the contour of the shaft (30) when the transmission adapter (60) is uncoupled from a proximal end (51) of a transmission element (50).

10. Medical instrument (10) with:
a handling device (20) with a first part (21), and a second part (22) which is movable relative to the first part (21);
a shaft (30) with a proximal end (31), which can be coupled mechanically to the first part (21) of the handling device (20), and a distal end (32);
a transmission element (50), movable in the shaft (30), for transmitting at least one of a force and a torque between the second part (21) of the handling device (20) and
the distal end (32) of the shaft (30);
a transmission adapter (60) according to one of Claims 1 to 7.

11. Medical instrument (10) according to the preceding claim, wherein the transmission adapter (60) is a component part of a shaft (30) according to Claim 8 or 9.

12. Medical instrument (10) according to one of Claims 10 and 11, with:
a plurality of alternatively usable transmission elements (50) with differently designed proximal ends (51);
a plurality of alternatively usable transmission adapters (60),
wherein the first coupling elements (61) of all the transmission adapters (60) are identical and designed for releasable mechanical coupling to the second part (22) of the handling device (20),
wherein the second coupling elements (68) of the plurality of transmission adapters (60) are designed differently,
wherein each transmission adapter (60) has a second coupling element (68) which is designed for releasable mechanical coupling to a proximal end (51) of one of the transmission elements (50).

## Revendications

1. Adaptateur de transfert (60) pour un instrument médical (10) comprenant une tige (30), un élément de transfert (50) déplaçable dans la tige (30) et un dispositif de manipulation (20) comprenant une première partie (21) qui peut être accouplée mécaniquement rigidement à une extrémité proximale (31) de la tige (30) et une deuxième partie (22) qui est mobile par rapport à la première partie (21), l'adaptateur de transfert (60) présentant :
un premier accouplement (61) pour l'accouplement mécanique amovible à la deuxième partie (22) du dispositif de manipulation (20) ;
un deuxième accouplement (68) pour l'accouplement mécanique positif amovible à une extrémité proximale (51) de l'élément de transfert (50),
**caractérisé en ce que** l'adaptateur de transfert (60) est intégré dans une région proximale de la tige (30) de sorte qu'il ne peut pas être retiré de la tige de manière non destructive ou sans l'utilisation d'outils.

2. Adaptateur de transfert (60) selon la revendication précédente, dans lequel le deuxième accouplement (68) comprend une mâchoire de préhension mobile (81, 82) pour retenir l'extrémité proximale (51) de l'élément de transfert (50) par engagement par correspondance de formes ou par force.

3. Adaptateur de transfert (60) selon la revendication précédente, dans lequel la mâchoire de préhension mobile (81, 82) peut pivoter autour d'un axe de pivotement (88).

4. Adaptateur de transfert (60) selon la revendication 2, dans lequel la mâchoire de préhension mobile (81, 82) peut être déplacée le long d'une trajectoire prédéterminée.

5. Adaptateur de transfert (60) selon l'une quelconque des revendications 2 et 3, comprenant en outre :
un élément élastique (87) qui est prévu et réalisé de manière à précontraindre mécaniquement la mâchoire de préhension mobile (81, 82) vers une position saisissant l'extrémité proximale (51) de l'élément de transfert (50).

6. Adaptateur de transfert (60) selon l'une quelconque des revendications précédentes, dans lequel le deuxième accouplement (68) est réalisé de manière à être verrouillé lorsque l'adaptateur de transfert (60) est disposé dans un dispositif de manipulation (20).

7. Adaptateur de transfert (60) selon l'une quelconque des revendications précédentes, dans lequel le premier accouplement (61) de l'adaptateur de transfert (60) présente une portion de surface ayant la forme d'une surface sphérique.

8. Tige (30) pour un instrument médical (10), comprenant :
une extrémité proximale (31) pour l'accouplement à une première partie (21) d'un dispositif de manipulation (20) ;
une extrémité distale (32) pour l'accouplement à un outil (40);
un adaptateur de transfert (60) selon l'une quelconque des revendications précédentes, qui est mobile par rapport à la tige (30).

9. Tige (30) selon la revendication 8, lorsqu'elle se rapporte à l'une des revendications 2 à 5, et comprenant en outre :
une ouverture latérale (38) dans la tige (30), la mâchoire de préhension mobile (81, 82) étant disposée au moins en partie dans l'ouverture latérale (38),
la mâchoire de préhension mobile (81, 82) étant réalisée de manière à être située complètement à l'intérieur du contour de la tige (30) lorsqu'une extrémité proximale (51) d'un élément de transfert (50) est retenue par engagement par correspondance de formes par la mâchoire de préhension mobile (81, 82), et de manière à faire saillie au-delà du contour de la tige (30) lors du désaccouplement de l'adaptateur de transfert (60) d'une extrémité proximale (51) d'un élément de transfert (50).

10. Instrument médical (10) comprenant :
un dispositif de manipulation (20) comprenant une première partie (21) et une deuxième partie (22), qui est mobile par rapport à la première partie (21) ;
une tige (30) avec une extrémité proximale (31) qui peut être accouplée mécaniquement à la première partie (21) du dispositif de manipulation (20) et une extrémité distale (32) ;
un élément de transfert (50) déplaçable dans la tige (30) pour le transfert d'au moins soit une force soit un couple entre la deuxième partie (21) du dispositif de manipulation (20) et l'extrémité distale (32) de la tige (30) ;
et un adaptateur de transfert (60) selon l'une quelconque des revendications 1 à 7.

11. Instrument médical (10) selon la revendication précédente, dans lequel l'adaptateur de transfert (60) fait partie d'une tige (30) selon la revendication 8 ou 9.

12. Instrument médical (10) selon l'une quelconque des revendications 10 et 11, comprenant :
une pluralité d'éléments de transfert (50) pouvant être utilisés de manière alternative avec des extrémités proximales (51) réalisées différemment ;
une pluralité d'adaptateurs de transfert (60) pouvant être utilisés de manière alternative, les premiers accouplements (61) de tous les adaptateurs de transfert (60) étant identiques et étant réalisés en vue de l'accouplement mécanique amovible à la deuxième partie (22) du dispositif de manipulation (20),
les deuxièmes accouplements (68) de la pluralité d'adaptateurs de transfert (60) étant réalisés de manière différente,
chaque adaptateur de transfert (60) présentant un deuxième accouplement (68) qui est réalisé en vue de l'accouplement mécanique amovible à une extrémité proximale (51) de l'un des éléments de transfert (50).
